# EUROPÄISCHE PATENTSCHRIFT

(11) **EP 0 672 407 B1**
(45) Veröffentlichungstag und Bekanntmachung des Hinweises auf die Patenterteilung: **15.05.2002**
(21) Anmeldenummer: 95102954.5
(22) Anmeldetag: 02.03.1995
(51) Int. Cl.: A61K 7/08

(54) **Verwendung der Diacetylweinsäureester von Fettsäureglyceriden als Haarkonditioniermittel**
Use of diacetyl tartaric acid esters of fatty acid grycerides as hair conditioner
Utilisation des esters de l'acide diacétyltartarique de glycérides d'acides gras en tant qu'agents de conditionnement de cheveux

(30) Priorität: 15.03.1994 DE 4408668
(43) Veröffentlichungstag der Anmeldung: 20.09.1995
(73) Patentinhaber: Goldschmidt AG, 45127 Essen (DE)
(72) Erfinder: Leidreiter, Holger, Dr., D-45529 Hattingen (DE); Müller, Felix, Dr., D-45277 Essen (DE)

(56) Entgegenhaltungen:
- DE-C- 2 021 565
- US-A- 2 236 516

## Beschreibung

Die Erfindung betrifft die Verwendung der Diacetylweinsäureester von Fettsäureglyceriden als Haarkonditioniermittel in Haarpflegemitteln mit verbesserten Konditioniereigenschaften.

Es ist bekannt, daß moderne Haarbehandlungen, wie Dauerwellen, Bleichen, Tönen, Färben, Shampoonieren, die natürliche Haarbeschaffenheit beeinträchtigen können mit der Folge, daß das Haar trocken, spröde und schwierig zu handhaben ist. Dies kann einmal hervorgerufen werden durch Entfettung des Haares und der Kopfhaut, zum andern können Haarfärbemittel und alkalische Medien, wie Dauerwellen, die Haarstruktur ganz entscheidend verändern. Die Oberfläche wird stumpfer und die gesamte Struktur von Cortex und Cuticula wird so geschwächt, daß das Haar brüchig wird und nur noch geringen Dehnungskräften widersteht. Derartige Haarschädigungen werden auch durch natürliche Alterung, Lichtbleichung, häufiges Waschen und mechanische Abnutzung hervorgerufen.

Zur Verminderung dieser Effekte werden daher kosmetischen Haarbehandlungsmitteln, wie Shampoos und Spülungen, Haarkonditioniermittel zugesetzt. Ziel ist dabei die Strukturverbesserung des Haares und damit die Verbesserung der Kämmbarkeit und des Griffes des nassen Haares sowie eine Reduzierung der Verknotung direkt nach der Anwendung. Das trockene Haar soll Glanz aufweisen sowie ebenfalls gute Griff- und Kämmbarkeitseigenschaften besitzen. Ebenso wird eine Verminderung der elektrostatischen Aufladung angestrebt.

Als Haarkonditioniermittel werden im Stand der Technik zahlreiche Mittel unterschiedlichster Art vorgeschlagen, wie Acryl-, Styrol-, Vinyloder Siliconderivate, Produkte aus Naturstoffen, wie Pflanzenextrakte oder derivatisierte Produkte und tierische oder pflanzliche Fettprodukte.

Es hat sich jedoch als schwierig erwiesen, möglichst alle der genannten Pflegeeffekte auf einem hohen Niveau zu erreichen.

Aufgabe der vorliegenden Erfindung war daher die Bereitstellung eines Haarkonditioniermittels mit einem möglichst breiten Spektrum an konditionierenden und pflegenden Eigenschaften von hoher Qualität.

Die Aufgabe wird gelöst durch die Verwendung der Diacetylweinsäureester von Fettsäureglyceriden der allgemeinen Formel 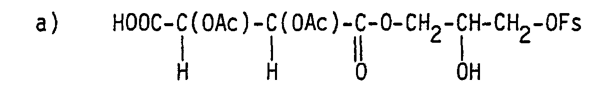 und 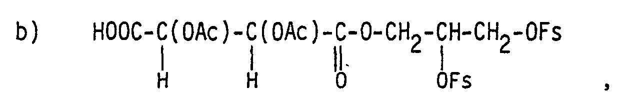 wobei
- Ac: der Acetylrest und
- Fs: der Acylrest einer Fettsäure oder eines Fettsäuregemisches mit 8 bis 18 C-Atomen ist,
wobei a) und b) im Gewichtsverhältnis 100 : 0 bis 60 : 40 vorliegen, als Haarkonditioniermittel in Zubereitungen für die Haarpflege.

Zur Herstellung der Diacetylweinsäureester werden bevorzugt die Monound Diglyceride linearer Fettsäuren mit 8 bis 18 C-Atomen eingesetzt, insbesondere der aus natürlichen Fetten gewonnenen, ggf. gehärteten Fettsäuren und Fettsäuregemische mit 16 bis 18 C-Atomen. Als Beispiele seien genannt Palmitinsäure, Stearinsäure, ölsäure, Linolsäure und Linolensäure.

In gleicher Weise ist auch die technisch herstellbare Isostearinsäure einsetzbar.

Bevorzugt werden dabei die destillierten Fettsäuremonoglyceride eingesetzt, da mit Zunahme des Diglyceridanteils die entsprechend erhaltenen Diacetylweinsäureester mit ihrer höheren Hydrophobie Löslichkeitsprobleme bei der Herstellung der Konditionierformulierungen hervorrufen können.

Als Weinsäure sind alle optischen Isomeren in gleicher Weise einsetzbar; sinnvollerweise wird man bevorzugt die natürlich vorkommende 2R,3R-Weinsäure verwenden.

Die Herstellung der erfindungsgemäß verwendeten Ester erfolgt in bekannter Weise, wie sie z.B. in DE-C-20 21 565 bzw. in ZFL Zeitschrift für Lebensmittel-Technologie und Verfahrenstechnik, 31. Jahrgang 1980, Heft 6, beschrieben ist. Bedingt durch das Herstellungsverfahren und den Einsatz technischer Produkte können dabei Nebenprodukte, wie Didiacetylweinsäure-monoglyceride oder Mono-diacetylweinsäure-monoessigsäure-monoglyceride, im gewünschten Produkt enthalten sein.

Es können beispielsweise Diacetylweinsäureester auf Basis von Glycerinstearat (Diacetylweinsäureester A) oder auf Basis Glycerinlaurat (Diacetylweinsäureester B) hergestellt werden. Dabei können diese Produkte zur besseren Handhabbarkeit auch in verdünnter oder compoundierter Form eingesetzt werden. Dafür kommen alle in der Kosmetik üblichen Zusatzstoffe in Frage. Es können Lösungsmittel, wie Alkohole, Glykole, Öle oder auch Wasser eingesetzt werden. Die Diacetylweinsäureester können gelöst oder dispergiert in diesen Compounds vorliegen. Im letzteren Fall ist ein oder mehrere Dispergiermittel zur Herstellung und Stabilisierung der Dispersion als Zusatzstoff notwendig. Insbesondere ist die Kombination mit anderen Konditioniermitteln, wie organischen Polymeren und/oder Siliconderivaten vorteilhaft, da solche Kombinationen erfahrungsgemäß synergistisch wirken können.

Die erfindungsgemäß verwendeten Diacetylweinsäureester können in verschiedenen Zubereitungsformen eingesetzt werden. Als Pflegezusatz in einer Haarspülung können sie problemlos mit oberflächenaktiven Substanzen in Form einer wäßrigen Zubereitung kombiniert werden. Besonders geeignet sind dabei quaternäre Ammoniumsalze.

Eine solche Formulierung zur Nachbehandlung gewaschener Haare kann folgendermaßen aufgebaut sein:

### Beispiel A (Haarspülung für leicht strapaziertes, normales Haar):

| | |
|---|---|
| Cetyl/Stearylalkohol mit 25 Mol Ethylenoxid | 1,5 % |
| Glycerinmono/distearat | 1,5 % |
| Cetylalkohol | 2,0 % |
| Diacetylweinsäureester B | 1,0 % |
| polyethermodifiziertes Polydimethylsiloxan | 0,4 % |
| Glycerin | 2,0 % |
| Citronensäure | 0,2 % |
| Wasser | 91,4 % |
| zuzüglich Farb- und Konservierungsstoffe | |

oder

### Beispiel B (Haarkur für trockenes, feines Haar):

| | |
|---|---|
| Cetyl/Stearylalkohol mit 25 Mol Ethylenoxid | 1,5 % |
| Glycerinmono/distearat | 1,5 % |
| Cetylalkohol | 2,0 % |
| alkylmodifiziertes Polydimethylsiloxan | 0,4 % |
| Diacetylweinsäureester B | 1,0 % |
| polyethermodifiziertes Polydimethylsiloxan | 0,4 % |
| mit quaternären Ammoniumgruppen modifiziertes Polydimethylsiloxan | 2,0 % |
| Glycerin | 2,0 % |
| Citronensäure | 0,2 % |
| Wasser | 88,0 % |
| zuzüglich Farb- und Konservierungsstoffe | |

Üblicherweise werden solchen Zubereitungen verschiedene Additive, wie öle, Fettsäuren und deren Ester, nichtionische oder amphotere oberflächenaktive Substanzen, Desinfektionsmittel, Parfüme, Farb- und Trägerstoffe usw. beigefügt.

In gleicher Weise können die erfindungsgemäß verwendeten Diacetylweinsäureester Shampoo-Formulierungen auf der Basis üblicher oberflächenaktiver Agentien zugegeben werden.

Derartige Formulierungen beruhen in der Regel auf
- mindestens einem waschaktiven Tensid (zumeist ein oder zwei anionische, ein amphoteres oder zwitterionisches sowie nichtionische Tenside),
- tensidischen niedermolekularen oder polymeren Additiven zur Einstellung des rheologischen Verhaltens,
- Perlglanz oder Trübung gebenden Zusatzstoffen,
- kationischen, anionischen und/oder nichtionischen Konditioniermitteln,
- Hilfsstoffen, wie Farbstoffen, Konservierungsmitteln, Säuren, Basen, Komplexierungsmitteln und
- Wasser.

Die Diacetylweinsäureester von Fettsäureglyceriden werden dabei in Mengen von 0,1 bis 10 Gew.-% eingesetzt.

### Beispiel C (Konditioniershampoo für normales Haar):

| | |
|---|---|
| Diacetylweinsäureester A | 2,0 % |
| Glycerinmonococoate mit 7 Mol Ethylenoxid | 3,0 % |
| Natriumlaurylethersulfat 2,5 Mol Ethylenoxid 28 %ig ¹⁾ | 40,0 % |
| Wasser | 43,2 % |
| Cocosfettsäureamidopropylbetain 38 %ig | 10,0 % |
| Glycerin Cocoat/01eat mit 18 Mol Ethylenoxid | 1,3 % |
| NaCl | 0,5 % |
| zuzüglich Farb- und Konservierungsstoffe | |

| | |
|---|---|
| 1) im Handel erhältlich z.B. unter der Bezeichnung Texapon N25 (Henkel, Düsseldorf) | |

### Beispiel D (Konditioniershampoo für trockenes Haar):

| | |
|---|---|
| Diacetylweinsäureester B | 1,1 % |
| kationisches Guar (Galaktomannan) | 0,3 % |
| Glycerinlaurat mit 30 Mol Ethylenoxid | 3,0 % |
| Natriumlaurylethersulfat 2,5 Mol Ethylenoxid 28 %ig ¹⁾ | 20,0 % |
| Alkylpolyglucosid mit C₁₂- bis C₁₈-Alkylgruppen ²⁾ | 12,0 % |
| Wasser | 49,2 % |
| mit quaternären Ammoniumgruppen modifiziertes Polydimethylsiloxan | 0,5 % |
| Cocosfettsäureamidopropylbetain 38 %ig | 9,0 % |
| fließfähige Perlglanzdispersion auf Basis von Ethylenglycoldistearat | 3,0 % |
| Glycerin Cocoat/Oleat mit 18 Mol Ethylenoxid | 0,9 % |
| NaCl | 1,0 % |
| zuzüglich Farb- und Konservierungsstoffe | |

| | |
|---|---|
| 1) im Handel erhältlich z.B. unter der Bezeichnung Texapon N25 (Henkel, Düsseldorf) | |
| 2) im Handel erhältlich z.B. unter der Bezeichnung Plantaren 1200 (Henkel, Düsseldorf) | |

### Beispiel E (Konditioniershampoo für empfindliche Kopfhaut):

| | |
|---|---|
| Diacetylweinsäureester A | 1,0 % |
| Glycerinmonococoate mit 7 Mol Ethylenoxid | 1,0 % |
| Glycerinlaurat mit 30 Mol Ethylenoxid | 3,0 % |
| polyethermodifiziertes Polydimethylsiloxan | 0,5 % |
| nichtionisches, polymeres Verdickungsmittel auf Basis von Polyacrylat-Copolymeren | 1,5 % |
| Laurylethersulfosuccinat 40 %ig ³⁾ | 10,0 % |
| Natriumlaurylethersulfat 2,5 Mol Ethylenoxid 28 %ig ¹⁾ | 12,0 % |
| Wasser | 51,0 % |
| Cocosfettsäureamidopropylbetain 38 %ig | 20,0 % |
| zuzüglich Farb- und Konservierungsstoffe | |

| | |
|---|---|
| 1) im Handel erhältlich z.B. unter der Bezeichnung Texapon N25 (Henkel, Düsseldorf) | |
| 3) im Handel erhältlich z.B. unter der Bezeichnung Elfanol 616 (Akzo, Düren) | |

Der Diacetylweinsäureester A ist in Tensidformulierungen klar löslich und beeinflußt die Schaumhöhe daher nicht. Der Diacetylweinsäureester B ist in Tensidformulierungen in der Regel nicht klar löslich und ergibt eine leichte Schaumverdichtung, zeigt aber effektivere pflegende und konditionierende Eigenschaften als der Diacetylweinsäureester A.

Die erfindungsgemäß verwendeten Diacetylweinsäureester zeichnen sich durch eine Reihe von ausgezeichneten Konditionierungseigenschaften aus.

So weist das damit behandelte Haar im nassen Zustand sowohl eine gute Kämmbarkeit als auch gute Griffeigenschaften auf. Die Neigung zur Verknotung ist dabei gering. Auch bei trockenem Haar werden gute Kämmund Griffeigenschaften erzielt. Nach dem Trocknen erhält das Haar einen Glanzeffekt, und die elektrische Aufladung wird verringert.

Die erfindungsgemäße Verwendung von Diacetylweinsäureestern als Konditioniermittel ist dabei für alle Haararten gleichermaßen geeignet. Besonders deutlich wird der Vorteil der erfindungsgemäßen Verwendung von Diacetylweinsäureestern bei der Beurteilung von Haar im Zustand nach der Trocknung. Besonders bevorzugt ist die Verwendung bei trockenem (im Sinne von wenig fettigem) und feinem Haar, da hier das fehlende natürliche Sebum der Kopfhaut und die mangelnde Festigkeit der Haare hervorragend ausgeglichen werden können. An normalem oder fettigem Haar wird jedoch kein negativer Überfettungseffekt beobachtet. Dies könnte darauf zurückzuführen sein, daß Diacetylweinsäureester weniger gegenüber fetthaltigem Haar und stärker gegenüber den Oberflächenproteinen fettfreier Haare substantiv ist.

Es ist hervorzuheben, daß gemäß der Erfindung Konditioniermittel bereitgestellt werden, die das ganze Spektrum der Pflegeeigenschaften abdecken. So wird beispielsweise die Kämmbarkeit der Haare nach dem Trocknen zwar verbessert, aber deren Frisierbarkeit und Griff nicht negativ beeinflußt. Dies ist bei Konditioniermitteln des Standes der Technik nicht der Fall, da diese bei einer Kämmbarkeitsverbesserung die Gleitfähigkeit der Haare erhöhen und somit die Haltbarkeit einer Frisur verringern und den Griff der Haare negativ beeinflussen.

In vergleichenden Versuchen mit Konditioniershampoos an Strähnen aus indoeuropäischem Humanhaar wurden die oben beschriebenen Eigenschaften gefunden.

| Beispiel | F | G | H | I |
|---|---|---|---|---|
| Diacetylweinsäureester B | 2,0 % | - | - | - |
| quaternisierte Cellulose | - | 2,0 % | - | - |
| kationisches Guar (Galaktomannan) | - | - | 2,0 % | - |
| mit quaternären Ammoniumgruppen modifiziertes Polydimethylsiloxan | - | - | - | 2,0 % |
| Glycerinmonococoate mit 7 Mol Ethylenoxid | 3,0 % | 3,0 % | 3,0 % | 3,0 % |
| Natriumlaurylethersulfat 2,5 Mol Ethylenoxid 28 %ig | 40,0 % | 40,0 % | 40,0 % | 40,0 % |
| Wasser | 43,2 % | 43,2 % | 43,2 % | 43,2 % |
| Cocosfettsäureamidopropylbetain 38 %ig | 10,0 % | 10,0 % | 10,0 % | 10,0 % |
| Glycerin Cocoat/Oleat mit 18 Mol Ethylenoxid | 1,3 % | 1,3 % | 1,3 % | 1,3 % |
| NaCl | 0,5 % | 0,5 % | 0,5 % | 0,5 % |

In den Vergleichstests werden Noten von 0 bis 4 vergeben, wobei 0 die schlechteste (mangelhaft) und 4 die beste (sehr gut) Bewertung ist. Bei Vergleich der vier Shampoos F, G, H und I miteinander werden die folgenden Noten gemittelt über zehn Probanden:

| Beispiel | F | G | H | I |
|---|---|---|---|---|
| im nassen Haar: | | | | |
| Verknotung | 2,2 | 1,5 | 1,2 | 2,6 |
| Kämmbarkeit | 2,2 | 1,6 | 1,2 | 2,4 |
| Griff | 2,2 | 1,9 | 1,2 | 2,2 |

| im getrockneten Haar: | | | | |
|---|---|---|---|---|
| Kämmbarkeit | 3,4 | 2,2 | 1,6 | 2,6 |
| Griff | 3,2 | 2,2 | 2,2 | 2,4 |
| Glanz | 2,2 | 2,0 | 2,0 | 1,8 |

In diesem Vergleich wird lediglich bei der Beurteilung der nassen Haare, die mit dem kationischen Siliconderivat behandelt worden sind, eine geringfügig bessere Bewertung erzielt. In allen anderen Vergleichen werden mit der erfindungsgemäßen Verwendung von Diacetylweinsäureestern bessere Ergebnisse erzielt.

Wie bereits ausgeführt, kann zur Erzielung einer darüber hinaus verbesserten und in ihrem Spektrum erweiterten Konditionierwirkung eine Kombination eines oder mehrerer verschiedener Konditioniermittel mit den Diacetylweinsäureestern eingesetzt werden.

Aufgrund der bekannten Verwendung der Diacetylweinsäureester von Fettsäuremonoglyceriden als Backhilfsmittel (DE-C-20 21 565) ist überdies die physiologische Verträglichkeit der erfindungsgemäß erhaltenen Haarkonditionierungsmittel völlig unproblematisch.

## Patentansprüche

1. Verwendung der Diacetylweinsäureester von Fettsäureglyceriden der allgemeinen Formel 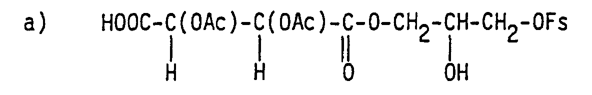 und 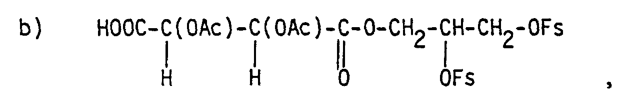 wobei
Ac der Acetylrest und
Fs der Acylrest einer Fettsäure oder eines Fettsäuregemisches mit 8 bis 18 C-Atomen ist,
wobei a) und b) im Gewichtsverhältnis 100 : 0 bis 60 : 40 vorliegen, als Haarkonditioniermittel in Zubereitungen für die Haarpflege.

## Claims

1. Use of diacetyltartaric esters of fatty acid glycerides of the general formula 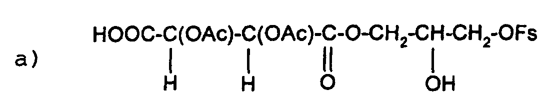 and 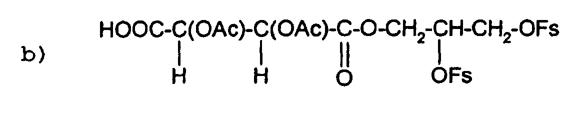 where
Ac is the acetyl radical and
Fs is the acyl radical of a fatty acid or of a fatty acid mixture having 8 to 18 carbon atoms,
where a) and b) are in the weight ratio 100:0 to 60:40, as hair-conditioning agents in haircare preparations.

## Revendications

1. Utilisation des esters de l'acide diacétyltartrique de glycérides d'acides gras de formule générale 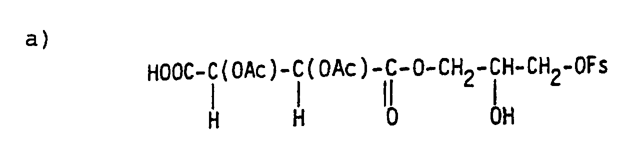 et 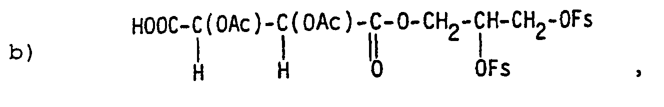 Ac étant le radical acétyle et
Fs étant le radical acyle d'un acide gras ou d'un mélange d'acides gras comprenant 8 à 18 atomes de carbone,
a) et b) se trouvant dans un rapport pondéral de 100:0 à 60:40,
comme agents de conditionnement des cheveux dans des préparations pour les soins capillaires.
